# EUROPEAN PATENT SPECIFICATION

(11) **EP 2 560 613 B1**
(45) Date of publication and mention of the grant of the patent: **15.10.2014**
(21) Application number: 11764289.2
(22) Date of filing: 20.04.2011
(51) Int. Cl.: A61K 9/00, A61K 9/16, A61K 9/50

(54) **FEXOFENADINE-BASED COMPOSITION AND PREPARATION PROCESS THEREFOR**
ZUSAMMENSETZUNG AUF FEXOFENADINBASIS UND HERSTELLUNGSVERFAHREN DAFÜR
COMPOSITION À BASE DE FEXOFÉNADINE ET SA MÉTHODE DE PRÉPARATION

(30) Priority: 21.04.2010 FR 1053034
(43) Date of publication of application: 27.02.2013
(73) Proprietor: SANOFI, 75008 Paris (FR)
(72) Inventor: LESOT, Axelle, F-75013 Paris (FR); LAMOUREUX, Gaël, F-75013 Paris (FR)
(74) Representative: Taravella, Brigitte
(86) International application number: PCT/IB2011/002000
(87) International publication number: WO 2011/151733

(56) References cited:
- WO-A1-97/18798
- WO-A2-2005/062722
- FR-A1- 2 753 904
- FR-A1- 2 857 263
- US-A- 4 948 622
- US-A- 5 891 476
- FAHAM A ET AL: "HOT MELT COATING TECHNOLOGY: INFLUENCE OF COMPRITOL(R) 888 ATO AND GRANULE SIZE ON CHLOROQUINE RELEASE", DIE PHARMAZIE, GOVI VERLAG PHARMAZEUTISCHER VERLAG GMBH, ESCHBORN, DE, vol. 55, no. 6, 1 June 2000 (2000-06-01), pages 444-448, XP000932193, ISSN: 0031-7144
- JOZWIAKOWSKI M J ET AL: "CHARACTERIZATION OF A HOT-MELT FLUID BED COATING PROCESS FOR FINE GRANULES", PHARMACEUTICAL RESEARCH, KLUWER ACADEMIC PUBLISHERS, NEW YORK, NY, US LNKD- DOI:10.1023/A:1015972007342, vol. 7, no. 11, 1 November 1990 (1990-11-01), pages 1119-1126, XP002052859, ISSN: 0724-8741

## Description

### FIELD OF THE INVENTION

The present invention relates to fexofenadine granules, to a composition containing them and to a fexofenadine hot melt coating process. The invention also relates to a formulation comprising a fexofenadine composition.

### PRIOR ART AND TECHNICAL PROBLEM

Fexofenadine is characterized by organoleptic or physicochemical properties, in particular its strong bitterness that it is desirable to mask. The process of hot-melt coating of fexofenadine thus allows efficient masking of its bitter taste without, however, unacceptably slowing down its dissolution.

In the field of masking the taste of active principles that have an unacceptable taste, many tests, using conventional technologies, have been performed: granulation, coating in a fluidized air bed, etc. The excipients tested were composed of polymers: mainly cellulose derivatives (HPC, HPMC, EC) and methacrylic acid derivatives (Eudragit range) and polyvinyl acetate derivatives. These tests often came up against insufficient taste masking.

In this respect, patent EP 1458387 describes orodispersible tablets comprising fexofenadine granules comprising a mixture of particular excipients. The described granules are coated granules containing fexofenadine microcrystals and binders. These granules are obtained by simple granulation of the active principle with binders, such as methacrylic acid derivatives.

Patent US 6113942 describes tablets comprising fexofenadine in the form of granules. The granules are obtained by mixing various components, followed by simple granulation.

Moreover, more innovative technologies have also been used in the context of taste masking, such as microencapsulation via a process in supercritical CO₂ phase or via a simple or complex coacervation method. The same masking polymers, mentioned above, are thus found, along with less conventional excipients such as chitosan, alginates and gelatin. The major drawbacks encountered in these technologies are the industrial implementation and feasibility.

In the same approach, spray-drying and spray-cooling processes were tested.

This latter technique, of "spray-cooling", described in WO 2004/058 137 consists in melting a fatty matrix and in incorporating the active principle therein. This active principle is either dissolved or dispersed in the fatty phase, composed of fatty acids, a mixture or esters of fatty acids, of fatty alcohols or of waxes.

The molten mixture is then conveyed, via a peristaltic pump, to a two-fluid nozzle where it is sprayed as more or less fine droplets (depending on the applied parameters). The droplets are finally cooled with a stream of cold air in the spraying chamber and converted into solid granules containing the active principle dispersed throughout the fatty matrix.

Although this spray-cooling process makes it possible to obtain pharmaceutical compositions with satisfactory taste masking, while at the same time ensuring dissolution of the active principle that is not slowed down in acidic medium, there nevertheless remains an unsolved problem of physical heat stability of the product obtained. Similarly, this process remains incompatible with active principles that are by nature heat-sensitive.

Furthermore, in the context of this spray-cooling process, the concentration of active principle in the composition is limited by the viscosity of the solution or suspension to be sprayed, which greatly reduces the active principle content of the final composition. Only compositions with a low dose of active principle can be obtained, i.e. compositions comprising an amount of active principle not exceeding 30% by weight in the final composition.

Moreover, the use of a hot-melt coating process was described in WO 02/07706**.** The said document describes the use of this technology for coating heat-sensitive active principles in the form of solid particles that are not granulated beforehand. The targeted coating amounts are low and do not allow satisfactory masking of the taste of very unpleasant active principles.

As a result, the use of the hot-melt coating process described in the said document is not suitable for obtaining sufficiently effective masking of the taste and odour of an active principle characterized by strong bitterness and a strong odour, especially masking that is sufficient to permit ingestion of the medicament by a child.

The Applicant has now found a novel improved process for solving these drawbacks and in particular for obtaining a fexofenadine composition in the form of coated granules that mask the bitter taste of fexofenadine.

### SUMMARY OF THE INVENTION

The aim of the invention is to propose a fexofenadine composition in the form of coated granules and a process for preparing the said composition, which solve at least the drawbacks mentioned above.

The problem solved by this manufacturing method, which comprises formulation with the aid of fatty matrices, is that of masking of the taste of fexofenadine, while at the same time obtaining relatively rapid *in vitro* release of fexofenadine (for example a minimum release of 70% of the active principle in 60 minutes).

Furthermore, the maximum concentration of active principle that may be used in this process is not limited, the product thus obtained may be highly dosed, i.e. compositions that may comprise an amount of active principle exceeding 30% by weight in the final composition may be obtained. This makes it possible to appreciably reduce the doses of products to be administered to the patient, in particular to young children or to the elderly.

According to a first aspect, the invention relates to a fexofenadine composition comprising (a) a granule centre formed from fexofenadine grains aggregated in the presence of a binder and optionally of a diluent or lubricant, and (b) a layer for coating the said granule centre formed from a fatty matrix, in which composition:
- the fexofenadine represents at least 10%, preferably 15% or even 20% by weight relative to the weight of the composition; the fexofenadine represents not more than 90%, preferably not more than 60% or 50% by weight relative to the weight of the composition, or even less than 40% by weight relative to the weight of the composition, preferably the fexofenadine represents from 20% to 40% relative to the weight of the composition;
- the fatty matrix represents more than 10% by weight, preferably from 50% (inclusive) to 85% (inclusive) by weight of the composition, the fatty matrix optionally comprising an adjuvant, preferably chosen from hydrophilic agents, surfactants or mixtures thereof, and these adjuvants representing less than 10% by weight of the composition and preferably from 1% to 3% by weight relative to the weight of the composition;
- the binder, preferably a hydrophilic agent chosen from hydrophilic polymers and hot-melt agents, represents from 0.2% to 18% by weight relative to the weight of the composition, preferably the binder represents less than 18% by weight for a hot-melt agent, or even, preferably, the binder represents less than 10% by weight for a hydrophilic polymer relative to the weight of the composition;
- the diluent, if necessary, as filler represents a content of from 0 to 78.8%, preferably from 0 to 39% and preferably from 5% to 15% by weight relative to the weight of the composition;
- the lubricant, if necessary, as flow agent represents a content of from 0 to 1.8% relative to the weight of the composition.

The weight percentage is expressed relative to the weight of the fexofenadine composition in the form of coated granules, unless otherwise mentioned.

Preferably, the binder is a hydrophilic polymer, preferably chosen from the group of cellulose derivatives (hydroxypropylcellulose or ethylcellulose), povidone (polyvinylpyrrolidone or PVP), sucrose, gums, starches, gelatin, macrogols (polyethylene glycols) and a mixture thereof.

According to another mode, the binder is a hot-melt agent chosen from Macrogols (PEG), sucroesters or poloxamers, and represents approximately from 0.2% to 20% and preferably from 1% to 15% by weight relative to the amount of active principle to be granulated in step E1).

A hydrophilic polymer and in particular povidone is preferably used as binder.

According to one particular embodiment, the diluent is chosen from polyols, celluloses, sugars, lactoses, starches, kaolin, calcium phosphates, calcium or magnesium carbonates, or derivatives thereof. According to one particular embodiment, the diluent is chosen from starches, for instance corn starch. This diluent represents approximately from 0 to 39% and preferably from 5% to 15% by weight of the composition. The said diluent may optionally act as permeabilizer, thus facilitating the dissolution of fexofenadine.

Preferably, in the composition according to the invention, the said fatty matrix is formed from C14 to C22 and preferably C16 to C18 long carbon-chain saturated fatty acids, pure or as mixtures, and/or the corresponding fatty alcohols thereof, and the binder is chosen from hydrophilic polymers.

Preferably, the fatty matrix is formed from stearic acid, palmitic acid, myristic acid, pure or as mixtures, and/or the corresponding fatty alcohols thereof. According to one particular aspect, the fatty matrix is formed from stearic acid.

Preferably, the said fatty matrix comprises an adjuvant chosen from the group of surfactants (phospholipid, polysorbate, lauryl sulfate), hydrophilic excipients such as sucrose, polyols, cellulose, lactose, silica, dicalcium phosphate, carbonates, starch, macrogols, agents that are soluble at acidic pH (methacrylic derivatives) pure or as mixtures, preferably phospholipids. Preferably, the adjuvant is a glycerolipid, in particular a phospholipid. More preferentially, this phospholipid is a lecithin (phosphatidylcholine), preferably soya lecithin.

Preferably, the adjuvant is a surfactant that represents less than 10% by weight, preferably less than 5% by weight, preferably from 1% to 3%, or even from 1% to 2%, by weight relative to the weight of the composition.

Preferably, in the composition according to the invention, the binder is povidone and the diluent is chosen from starches and in particular corn starch.

Preferably, in the composition according to the invention, the fatty matrix is stearic acid and the adjuvant is soya lecithin. A composition according to the invention in which the binder is povidone, the diluent is chosen from starches, and in particular corn starch, the fatty matrix is stearic acid and the adjuvant is soya lecithin is particularly preferred.

Preferably, the amount of fexofenadine represents more than 10% by weight and ranges up to 90% by weight relative to the weight of the composition.

Preferably, the amount of fexofenadine represents more than 10% by weight of the composition and the fatty matrix comprises an adjuvant.

Preferably, the amount of fexofenadine represents from 20% to less than 40% by weight of the composition and the fatty matrix comprises at least one adjuvant.

Preferably, the fexofenadine composition according to the invention is obtained via the process as described below.

According to another subject, the invention relates to a pharmaceutical composition comprising the fexofenadine composition as described above or as may be prepared according to the process described below.

According to one particular subject, the invention relates to a sachet for a drinkable suspension or a sachet comprising a composition of granules to be swallowed (also referred to as powders to be swallowed), and in particular a single-dose sachet comprising a composition of granules to be swallowed, the said sachet comprising the fexofenadine composition as defined above in the presence of an excipient, chosen in particular from diluents, viscosity modifiers, sequestrants, buffers, preserving agents, lubricants, wetting agents, effervescent agents, dyes, sweeteners, salivating agents, flavourings, and a mixture thereof.

According to another subject, the invention relates to tablets, to be chewed, swallowed or sucked, or orodispersible tablets with masked taste comprising the fexofenadine composition as defined above in the presence of an excipient, in particular chosen from diluents, binders, lubricants, salivating agents, anaesthetics, wetting agents, preserving agents, disintegrants, effervescent agents, dyes, sweeteners, flavourings, and a mixture thereof.

According to another subject, the invention relates to the use of the fexofenadine composition as defined above for the preparation of sachets for a drinkable suspension, sachets comprising a composition of granules to be swallowed, and in particular single-dose sachets comprising a composition of granules to be swallowed, tablets to be chewed, tablets to be swallowed, tablets to be sucked, orodispersible tablets with masked taste.

The tablets or sachets according to the invention comprise the composition according to the invention and more specifically the coated fexofenadine granules as defined above, and an excipient, in particular as defined above.

According to one particular embodiment, the final formulation, for example in the form of tablets or sachets, may contain amounts of fexofenadine ranging from 20 to 200 mg (for instance 30, 60, 120 or 180 mg). The weight ratio of coated granules relative to the weight amount of excipient in the final formulation may vary within a wide range, for example from 0.2 to 0.8.

According to another subject, the invention relates to a process for preparing a fexofenadine composition as defined above, the said process comprising the steps of:
E1) preparation of the granule centre by spraying an aqueous solution, an organic solution (for example C1-C4 alcohol) or a mixture thereof (for instance an aqueous-alcoholic solution) comprising a binder or by spraying a molten binder onto the fexofenadine alone or as a mixture with a lubricant and/or preferably a diluent;
E2) coating by spraying onto the granules the said fatty matrix melted beforehand in a melting vessel at a temperature from about 10 to 20°C above its melting point;
E3) cooling the composition obtained.

According to one preferred mode, the process allows the preparation of a composition in which the fexofenadine represents at least 10% and not more than 90% and preferably from 20% to 50%, and the fatty matrix comprising an adjuvant represents more than 10% by weight and preferably from 50% and up to 85% by weight of the composition.

According to one preferred embodiment, the process allows the preparation of a composition in which the fexofenadine represents less than 40% by weight, preferably from 20% to 40%, advantageously from 30% to 40%, relative to the weight of the composition, and the fatty matrix represents more than 10% by weight, preferably from 50% and up to 85% by weight of the composition, advantageously from 50% to 60% (inclusive) by weight of the composition.

Preferably, the size of the coated granules obtained after step E3) is less than 500 µm, preferably less than 350 µm, preferably ranging from 50 to 350 µm.

Preferably, the particle size of the final product obtained after step E3) is distributed according to the following range:
- less than 15% by weight of the coated granules are greater than 500 µm;
- more than 80% by weight, preferably more than 90% by weight of the coated granules are between 350 and 50 µm; and
- less than 20% by weight, preferably less than 5% by weight of the coated granules are less than 50 µm.

Preferably, the aqueous or organic solution used in step E1 comprises, as binder, a hydrophilic polymer preferably chosen from the group of cellulose derivatives (hydroxypropylcellulose or ethylcellulose), povidone (polyvinylpyrrolidone or PVP), sucrose, gums, starches, gelatin, macrogols (polyethylene glycols), which represents approximately from 15% to 45% and preferably 20% to 40% by weight of the said solution.

Preferably, the binder used in step E1 for the granulation is a hot-melt agent chosen from Macrogols (PEG), sucroesters or poloxamers, and represents approximately from 0.2% to 20% and preferably from 1% to 15% by weight relative to the amount of active principle to be granulated in step E1).

A hydrophilic polymer and in particular povidone is preferably used as binder.

According to one particular embodiment, the aqueous or organic solution used in step E1 is sprayed onto the fexofenadine mixed with a diluent (filler). The diluents used in granulation to increase the charge to be granulated are preferably chosen from polyols, celluloses, sugars, lactoses, starches, kaolin, calcium phosphates, calcium or magnesium carbonates or derivatives thereof. According to one particular embodiment, the diluent is chosen from starches, for instance corn starch. This diluent represents approximately from 0 to 39% and preferably from 3% to 15% by weight of the composition. The said diluent may optionally act as permeabilizer, thus facilitating the dissolution of the fexofenadine.

Preferably, the fatty matrix is formed from C14 to C22 and preferably C16 to C18 long-carbon-chain saturated fatty acids, pure or as mixtures, and/or the corresponding fatty alcohols thereof.

Preferably, the fatty matrix is formed from stearic acid, palmitic acid, myristic acid, pure or as mixtures, and/or the corresponding fatty alcohols thereof. According to one particular aspect, the fatty matrix is formed from stearic acid.

Preferably, the adjuvant mixed with the fatty matrix is chosen from the group of surfactants (phospholipid, polysorbate, lauryl sulfate), hydrophilic excipients such as sucrose, polyols, cellulose, lactose, silica, dicalcium phosphate, carbonates, starch, macrogols, agents that are soluble at acidic pH (methacrylic derivatives) pure or as mixtures, preferably phospholipids. Preferably, the adjuvant used is a glycerolipid, in particular a phospholipid. More preferentially, this phospholipid is a lecithin (phosphatidylcholine), preferably soya lecithin.

Preferably, the weight percentage of the adjuvant added to the fatty substance in step E2 is less than 10% by weight, preferably less than 5% by weight, preferably ranging from 1% to 3%, or even from 1% to 2%, by weight relative to the weight of the composition.

Preferably, the weight percentage of the binder constituting the coating for the granule obtained in step E1 represents from 1% to 7% (or from 1% to 5%), more particularly from 4% to 7%, by weight relative to the weight of the composition for a hydrophilic polymer. According to one particular embodiment, the weight percentage of the binder constituting the coating for the granule obtained in step E1 represents from 0.2% to 18% for a hot-melt agent.

Preferably, the process is followed by a step E4 of formulation of the coated granules obtained in step E3 with excipients such as diluents, fillers, viscosity modifiers, disintegrants, dyes, sweeteners, salivating agents, flavourings, preserving agents, wetting agents, effervescent agents, lubricants, buffers or sequestrants, for the manufacture of an oral formulation in the form of a composition of granules to be swallowed for sachets, granules for a drinkable suspension, granules for tablets or granules for orodispersible tablets.

### DETAILED DESCRIPTION OF EMBODIMENTS OF THE INVENTION

### Process for preparing the fexofenadine composition

### 1) Granulation step

In a first step of the process, an aqueous or organic wetting solution comprising a binder, preferably of hydrophilic polymer type, is sprayed onto the fexofenadine in crude form or onto a fexofenadine/diluent mixture (filler) or onto a fexofenadine/lubricant mixture or a fexofenadine/diluent/lubricant mixture, preferably onto a fexofenadine/diluent mixture.

Preferably, the aqueous or organic solution contains from about 10% to 45% by weight, preferably 15% to 40% by weight and advantageously 15% of binder, preferably of hydrophilic polymer type, in the said solution.

The parameters used for this granulation step are adapted to the properties of fexofenadine and the equipment used.

This step makes it possible to obtain finely granulated fexofenadine by homogenization and recentring of the particle size distribution of fexofenadine, for the purpose of improving the quality of the second coating step.

According to one variant, calibration of the fexofenadine in the form of granules obtained in the above step may also be performed to select the granules with a diameter of less than 500 µm.

### 2) Coating step via the hot-melt process

In a second step, the fexofenadine in granulated form is preferably fluidized in a fluidized-air bed.

The fatty matrix as described above is melted, with stirring, in a melting vessel at a temperature about 10 to 20°C above its melting point.

The melting points of the fatty substances used are in the region of about 50 to 80°C, preferentially from 55 to 65°C or preferably about 60°C. This melting range was preferentially chosen, on the one hand for a question of physical stability of the composition thus formulated, and on the other hand so as to finally have the fastest possible release of fexofenadine.

According to one preferred embodiment, an adjuvant (hydrophilic excipient or surfactant) as described above is added to the melted fatty matrix, optionally with a preserving agent. This adjuvant promotes the generation of a rapid release profile for the composition.

The melted mixture is then sprayed onto the fexofenadine so as to produce a coating via the hot-melt coating process. The parameters applied during the coating process are summarized below.

The coating process may be performed in a fluidized-air bed apparatus.

The nozzles are fed, on the one hand, with the molten mixture that circulates via a pump in an entirely insulated circuit and traced, on the other hand, with hot compressed air fed in via an air heater.

The main operating parameters applied, which are adapted to the equipment used and to the batch size used, are as follows:
- T° of the molten fatty substance: from 70 to 95°C, depending on the properties and characteristics of the fatty substance;
- T° spraying compressed air: from 80 to 120°C;
- compressed air pressure: from 0.5 to 1.5 bar;
- T° of the feed circuits: between 80 and 100°C;
- liquid flow rate: from 5 to 30 g/min;
- air flow rate: this is usually adapted according to the size of the equipment. For apparatus with a working capacity of 3 kg, an air flow rate of 50 to 160 m³/h is generally used, depending on the filling charge of the equipment;
- T° inlet air: from 25 to 50°C, according to the properties of the fatty substance used.

After these steps and after cooling, a fexofenadine composition comprising a granule centre formed from fexofenadine grains aggregated in the presence of binder and a coating layer for the said granule centre, formed from fatty substance, is obtained.

### Description of the fexofenadine composition

### Fexofenadine

The invention relates to any type of fexofenadine in the form of solid particles intended to be coated to mask their unsatisfactory organoleptic or physicochemical properties, in particular its strong bitterness.

Fexofenadine (IUPAC name: (RS)-4-(1-hydroxy-4-(4-(hydroxydiphenylmethyl)-1-piperidyl)butyl)-α,α-dimethylbenzeneacetic acid) is a metabolite of terfenadine and is an antihistamine of rapid and prolonged action acting selectively on the peripheral H1 receptors. In hydrochloride form, it is used in the treatment of hay fever and similar allergic rhinites, and also in the treatment of chronic idiopathic urticaria.

### Active principle granulating agents

The granulating agents used in the first step of the process are binders preferably chosen from hydrophilic agents. They are used in a proportion of from 0.2% (or 1 %) to 18% by weight relative to the weight of the composition.

These hydrophilic agents are particularly chosen from the group of cellulose derivatives (hydroxypropylcellulose), povidone (polyvinylpyrrolidone), sucrose, gums, starches, gelatin, macrogols, sucroesters and poloxamers. Preferably, the binder is a hydrophilic polymer and in particular povidone.

According to one embodiment, hydrophilic polymers are especially used, preferably PEG or PVP in aqueous solution in a proportion of about 10% to 45% by weight in the aqueous solution.

Preferably, when the binder is a hydrophilic polymer, the weight percentage of the binder is less than 10% by weight, preferably less than 7% (or 5%) by weight, preferably ranging from 4% to 7% by weight relative to the weight of the composition.

According to another embodiment, hydrophilic agents chosen from hot-melt agents (melting point < 85°C), such as macrogols (PEG), sucroesters or poloxamers are used, especially during the use of moisture-sensitive active principles. The concentrations of hot-melt binders used are from about 0.2% to 20% and preferably from 1% to 15% by weight relative to the amount of active principle to be granulated.

Preferably, when the binder is a hot-melt agent, the weight percentage of the binder is less than 18% by weight, preferably less than 13.5% by weight relative to the weight of the composition, and preferably from 0.2% to 13.5% by weight relative to the weight of the composition.

The diluents used in granulation to increase the charge to be granulated or to facilitate the dissolution of the active principle are preferably chosen from polyols, celluloses, sugars, lactoses, starches, kaolin, calcium phosphates, calcium or magnesium carbonates, or derivatives thereof. According to one particular embodiment, the diluent is chosen from starches, for instance corn starch. These diluents are present in a proportion of between 0 and 80% of the weight of the granule, which represents 0 to 78.8% by weight relative to the weight of the composition. Preferably, these diluents are present in a proportion of between 0 and 39% and preferably from 3% to 15% by weight relative to the weight of the composition.

The lubricants used in granulation to improve the fluidization are preferably chosen from silicas and talc. These lubricants are present in a proportion of between 0 and 2% by weight of the granule, which represents 0 to 1.8% by weight relative to the weight of the final composition.

### Characteristics of fexofenadine in the form of granules

The granule comprising fexofenadine, the binder and optionally the diluent, obtained after the first granulation step has a mean particle size of less than 500 µm and preferably on average less than 200 µm.

### Fatty matrix

The fatty matrices are chosen from the group of C14 to C18 and preferably C16 to C18 long-carbon-chain saturated fatty acids, pure or as mixtures, and/or the corresponding fatty alcohols thereof and/or the corresponding esters of fatty acids and alcohols. Preferably, the fatty matrix is formed from stearic acid, palmitic acid, myristic acid, pure or as mixtures, and/or the corresponding fatty alcohols thereof. According to one aspect of the invention, the fatty matrix is formed from stearic acid.

Preferably, the fatty matrix represents more than 10% by weight, preferably more than 15% or more than 25% by weight or alternatively 50% or 60% by weight of the composition and up to 85% by weight of the composition.

According to one preferred embodiment, stearic acid is used as fatty matrix in a proportion of 50% or more, such as 60%, by weight relative to the weight of the composition, to improve the masking of the taste of the very unpleasant molecules.

### Fatty-matrix adjuvants

The adjuvants are chosen from the group of surfactants such as phospholipids, polysorbate, lauryl sulfate, hydrophilic excipients such as sucrose, polyols, cellulose, lactose, silica, dicalcium phosphate, starch, povidones, macrogols, agents that are soluble at acidic pH such as methacrylic derivatives; phospholipids are preferably used. Preferably, the adjuvant used is a glycerolipid, in particular a phospholipid. More preferentially, this phospholipid is a lecithin (phosphatidylcholine), preferably soya lecithin.

Preferably, the weight percentage of the adjuvant added to the fatty substance in the coating step is less than 10% by weight, preferably less than 5% by weight, preferably ranging from 0.5% to 3.5% or from 1% to 3% by weight relative to the weight of the composition.

According to one preferred embodiment, phospholipids, preferably lecithin (in particular soya lecithin), are preferably used as adjuvant, in a proportion of from 1% to 3%, or even from 1% to 2%, by weight relative to the weight of the composition, to improve their dissolution profile.

### Characteristics of the fexofenadine composition:

### Particle size

The particle sizes of the products resulting from this process are of the order of a few hundred microns (depending on the parameters applied and the type of equipment used).

The particle size distribution of the product obtained follows the following profile:
o less than 15% by weight of the coated granules are greater than 500 µm;
o more than 80% by weight and preferably more than 90% by weight of the coated granules are between 350 and 50 µm, and
o less than 20% by weight and preferably less than 5% by weight of the coated granules are less than 50 µm.

### Preferred amounts and ratio

The composition according to the invention is formed from fexofenadine comprising (a) a granule centre formed from fexofenadine grains aggregated in the presence of binder and (b) a coating layer for the said granule centre formed from fatty matrix.

In this composition, the preferred amounts and ratios of the various combinations of ingredients are represented below:
➢ fexofenadine represents at least 10%, preferably 15% or even 20% by weight relative to the weight of the composition; fexofenadine represents not more than 90% by weight, preferably not more than 60% or even not more than 50% by weight relative to the weight of the composition; according to one embodiment, fexofenadine represents less than 40% by weight relative to the weight of the composition, and fexofenadine preferably represents from 20% to less than 40% relative to the weight of the composition,
the fatty matrix represents more than 10% by weight, preferably from 50% to 85% by weight of the composition, preferably from 50% to 70% by weight of the composition,
the adjuvant, when it is present in the fatty matrix, represents less than 10% by weight of the composition, preferably from 1% to 3%, or even from 1% to 2%, by weight relative to the weight of the composition,
➢ the binder, preferably a hydrophilic polymer or a hot-melt agent, represents less than 18% (for a hot-melt agent) or even less than 5% (for a polymer) by weight relative to the weight of the composition,
➢ the diluent, if necessary, as filler represents a proportion of from 0 to 78.8% by weight relative to the weight of the composition,
➢ the lubricant, if necessary, as flow agent represents a proportion of from 0 to 1.8% by weight relative to the weight of the composition.

### Properties

The composition or the final formulation containing it allows efficient masking of the taste and odours of fexofenadine, which has unpleasant organoleptic properties. Specifically, the coating of fexofenadine allows the creation of a barrier around it so as to mask its bitter taste.

In parallel, products that show relatively rapid *in vitro* release of fexofenadine (for example a minimum release of 70% in 60 minutes) are obtained.

Moreover, by virtue of the effective masking of the taste, products with a high dose of fexofenadine may be obtained, for example containing more than 30% of fexofenadine, which makes it possible to appreciably reduce the doses of products to be administered to the patient, in particular to young children or the elderly.

### Evaluation of the taste masking:

The taste masking performed by this coating may be evaluated qualitatively, by means of taste tests performed by volunteers. The test consists of an oral administration of the product (tablets or composition of granules to be swallowed). The administration rules are as follows: do not take the test just after a meal (wait at least one hour), perform the test in a calm, quiet environment, drink water between each test, wait at least 2 minutes between each test, fill in an evaluation form. The evaluation consists in grading the disintegration time in the mouth, in specifying the mouthfeel (granular, pasty, etc.) and in specifying the perception of the bitterness and optionally in adding comments.

### in vitro dissolution/release:

The dissolution profiles of the composition obtained after the process are determined according to the following method. The dissolution is performed with paddles. The reagents used are: 0.001 M hydrochloric acid; acetonitrile and fexofenadine hydrochloride: reference substance.

The procedure is as follows:
➢ Temperature: 37°C ± 0.5°C
➢ Paddle speed: 50 rpm
➢ Dissolution medium: 0.001 M hydrochloric acid
➢ Dissolution volume: 900 ml

1 ml samples are collected on line in an HPLC vial at 5, 10, 15, 30, 45 and 60 minutes. 2.7 µm fibreglass filters are used. Two controls weighing 40.1 mg of reference substance (fexofenadine hydrochloride) are prepared in a 200 ml flask. The active principle is dissolved with 1 ml of acetonitrile and the volume is made up to the graduation mark with 0.001 N HCl. Analysis of the dissolution samples are performed by HPLC chromatography. Generally, the mean obtained corresponds to a mean of six tests performed. The dissolution samples are injected as collected in a vial.

The chromatographic conditions are as follows:
➢ Column: Luna C18 100 x 3.0 mm (diameter 3 µm)
➢ Flow rate: 0.3 ml/minute
➢ Wavelength: 220 nm
➢ Injection: 4 µl
➢ Column temperature: 30°C
➢ Mobile phase: 70V of 10 mM ammonium acetate and 30V of acetonitrile

### Formulation of the outer phase

The fexofenadine composition based on granules coated with fatty matrix according to the invention may then be incorporated into an external formulation for the manufacture of an oral form such as compositions of granules for a sachet to be placed directly in the mouth and to be swallowed with or without water, in particular compositions of granules to be swallowed for a single-dose sachet, granules for suspension, tablets for chewing, tablets for sucking, tablets for swallowing or orodispersible tablets.

In the case of formulation in sachets, the formulation of the outer phase may be enriched with surfactants or wetting agents so as to improve the resuspension of the granules obtained in aqueous medium.

Other excipients such as fillers or diluents, dyes, sweeteners, viscosity modifiers or gelling agents, adsorbents, buffers or flavourings, effervescent agents or salivating agents may also be added to the formulation for the purpose of improving the final aspect of the product.

The examples that follow illustrate the present invention without, however, limiting its scope. The percentages are given on a weight basis, unless otherwise mentioned.

### Examples

### Preparation of fexofenadine granules coated with a fatty matrix

### 1. Description of the hot-melt process for obtaining the compositions according to the invention

Fexofenadine and optionally corn starch are mixed together and fluidized in a fluidized-air bed. After a few minutes of fluidization at an average flow rate of 80 m³/h, spraying is started at an average flow rate of 20 g/min. The granulation solution is composed of water in which is dispersed povidone.

The inlet temperature is set at 65°C with an average flow rate of 20 g/min. Spraying lasts for about 1 hour. The grain is then dried in a fluidized-air bed with an inlet air flow rate of 80-100 m³/h and an inlet temperature of 65°C and an outlet temperature of about 45°C. The grain obtained is finely granulated and has a particle size predominantly less than 200 µm. Stirring is continued throughout the spraying.

The fatty matrix formed from stearic acid is melted at 80°C. After homogenization of the fatty substance, the phospholipid adjuvant is added to the molten fatty matrix to a proportion of 1% to 5% depending on the formulation, until homogenization of the mass is complete.

A few hundred grams of granulate are fluidized in the fluidized-air bed with an air flow rate of 70 to 90 m³/h (this flow rate is adapted as a function of the progress of the step, i.e. as a function of the charge and of the density, acquired gradually by the grain).

The inlet air temperature during coating is set at between 30° and 40°C. The molten mixture is then sprayed at a flow rate of 17 g/min on average. Once set, this flow rate remains constant throughout the spraying. The spraying pressures used are also kept constant and are between 0.5 and 0.9 bar. The spraying air used is heated to a target temperature of 90°C. Depending on the formula, the spraying time varies. Once the spraying is complete, the granule is cooled and then discharged.

### 2. Description of compositions according to the invention and results

Fexofenadine is granulated with 15% of PVP and coated with a proportion of 50% to 60% of stearic acid. Corn starch is added to certain formulae and the proportion of soya lecithin is also modified.
Formula 1: The proportion of soya lecithin is 5% in the coating solution. There is no addition of starch, and the proportion of stearic acid coating is 50%.
Formula 2: Starch is added to 5% in the coating solution (which corresponds to 10% of the amount of stearic acid). The proportion of soya lecithin is 1.5% and the proportion of stearic acid coating is 50%.
Formula 3: The proportion of soya lecithin is increased to 5% in the coating solution and the starch is at 5% in the coating solution (which corresponds to 10% of the amount of stearic acid). The proportion of stearic acid coating is 50%.
Formula 4: Starch is added to 10% in the coating solution (which corresponds to 20% of the amount of stearic acid). The proportion of soya lecithin is 1.5% and the proportion of stearic acid coating is 50%.
Formula 5: The proportion of starch is added to 5% to the fexofenadine for the granulation. The proportion of soya lecithin is 1.5% and the proportion of stearic acid coating is 50%.
Formula 6: The proportion of starch is added to 5% to the fexofenadine for the granulation. The proportion of soya lecithin is 1.5% and the proportion of stearic acid coating is 60%.

Table 1 collates formulae 1-4 and Table 2 presents formulae 5 and 6.

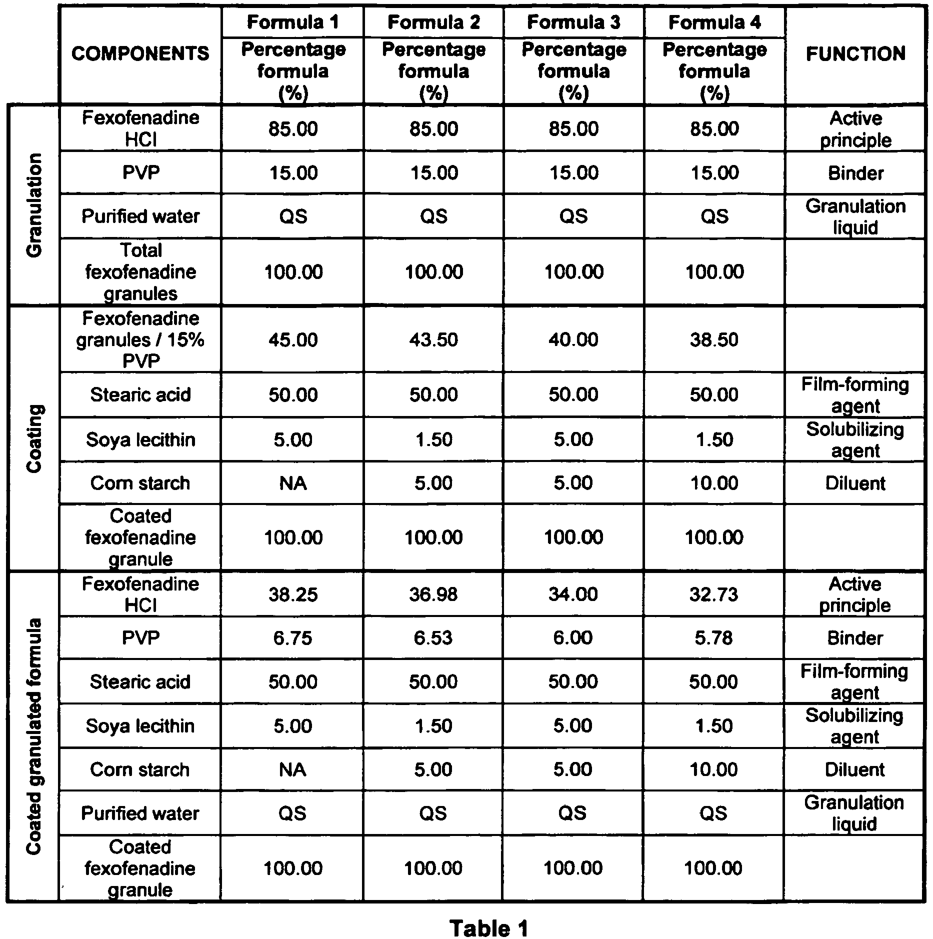

**Table 2**

| **COMPONENTS** | **Formula 5** | **Formula 6** | **FUNCTION** |
|---|---|---|---|
| | **Percentage formula (%)** | **Percentage formula (%)** | |
| Fexofenadine HCl | 76.24 | 76.24 | Active principle |
| PVP | 13.45 | 13.45 | Binder |
| Corn starch | 10.31 | 10.31 | Diluent |
| Purified water | QS | QS | Granulation liquid |
| **Total fexofenadine granules** | **100.00** | **100.00** | |
| Granules de fexofenadine / PVP | 48.50 | 38.50 | |
| Stearic acid | 50.00 | 60.00 | Film-forming agent |
| Soya lecithin | 1.50 | 1.50 | Solubilizing agent |
| **Coated fexofenadine granule** | **100.00** | **100.00** | |
| Fexofenadine HCl | 36.98 | 29.35 | Active principle |
| PVP | 6.52 | 5.18 | Binder |
| Stearic acid | 50.00 | 60.00 | Film-forming agent |
| Soya lecithin | 1.50 | 1.50 | Solubilizing agent |
| Corn starch | 5.00 | 3.97 | Diluent |
| Purified water | QS | QS | Granulation liquid |
| **Coated fexofenadine granule** | **100.00** | **100.00** | |

### 3. Pharmaceutical compositions according to the invention

The percentages indicated below are expressed as total weight of the composition.

### a) Preparation of granules or of a powder to be swallowed in a sachet:

| **COMPONENTS** | **PRODUCT 1** | | | | | **FUNCTION** |
|---|---|---|---|---|---|---|
| | **%** | **30 mg** | **60 mg** | **120 mg** | **180 mg** | |
| | **% Formula** | **Unit formula (mg)** | **Unit formula (mg)** | **Unit formula (mg)** | **Unit formula (mg)** | |
| Coated fexofenadine granules (formula 5) | **54** | 81 | 162 | 324 | 486 | Coated active principle |
| Mannitol | **17** | 25.5 | 51 | 102 | 153 | Diluent |
| Xylitol | **17** | 25.5 | 51 | 102 | 153 | Diluent |
| Sweetener | **2** | 3 | 6 | 12 | 18 | |
| Effervescent agents | **7** | 10.5 | 21 | 42 | 63 | |
| Flavourings | **2** | 3 | 6 | 12 | 18 | |
| Flow agent | **1** | 1.5 | 3 | 6 | 9 | |
| **TOTAL** | **100.0** | **150.0** | **300.0** | **600.0** | **900.0** | |

| **COMPONENTS** | **PRODUCT2** | | | | | **FUNCTION** |
|---|---|---|---|---|---|---|
| | **%** | **30 mg** | **60 mg** | **120 mg** | **180 mg** | |
| | **Percentage formula (%)** | **Unit formula (mg)** | **Unit formula (mg)** | **Unit formula (mg)** | **Unit formula (mg)** | |
| Coated fexofenadine granules (formula 6) | **68** | 102 | 204 | 408 | 612 | Coated active principle |
| Mannitol | **10.5** | 15.75 | 31.5 | 63 | 94.5 | Diluent |
| Xylitol | **10.5** | 15.75 | 31.5 | 63 | 94.5 | Diluent |
| Sweetener | **2** | 3 | 6 | 12 | 18 | |
| Effervescent agents | **6.5** | 9.75 | 19.5 | 39 | 58.5 | |
| Flavourings | **1.5** | 2.25 | 4.5 | 9 | 13.5 | |
| Flow agent | **1** | 1.5 | 3 | 6 | 9 | |
| **TOTAL** | **100.0** | **150.00** | **300.0** | **600.0** | **900.0** | |

The formulation of the external phase may vary especially in the following manner:
- Diluents: from 5% to 90%
- Flow agents: from 0 to 3%
- Viscosity modifiers: from 0 to 10%
- Effervescent agents: from 1% to 16%
- Sweeteners: from 0.5% to 5%
- Flavourings: from 0 to 5%
- Dyes: from 0 to 3%
- Buffers: from 0 to 3%
- Sequestrants: from 0 to 10%

The external phase is added to the coated granules manufactured according to the invention to a proportion of 20% to 80%.

All the excipients of the external phase, with the exception of the lubricants, are mixed with the coated granules. A lubrication phase is then optionally prepared before placing in the sachet.

### b) Preparation of tablets for chewing, swallowing or sucking:

The formulation of the external phase is as follows:
- Diluents: from 5% to 90%
- Lubricants: from 0.25% to 5%
- Sweeteners: from 0.2% to 5%
- Flavourings: from 0 to 5%
- Dyes: from 0 to 3%

The external phase is added to the coated granules manufactured according to the invention to a proportion of 20% to 80%.

All the excipients of the external phase are mixed with the coated granules. The whole is then tableted.

### c) Preparation of orodispersible tablets:

The formulation of the external phase is as follows:
- Diluents: from 5% to 90%
- Disintegrants: from 2% to 20%
- Salivating agents: from 0 to 5%
- Lubricants or flow agents: from 0.25% to 5%
- Sweeteners: from 0.2% to 5%
- Flavourings: from 0 to 5%
- Dyes: from 0 to 3%

The external phase is added to the coated granules manufactured according to the invention to a proportion of 20% to 80%.

All the excipients of the external phase are mixed with the coated granules. The whole is then tableted.

### d) Example of formulae of orodispersible fexofenadine tablets:

| **COMPONENTS** | **Percentage formula (%)** |
|---|---|
| Coated fexofenadine granule (formula 5) | **54** |
| Mannitol | **28** |
| Crospovidone (Kollidon CL) | **5** |
| Aspartame | **1.5** |
| Microcrystalline cellulose | **10** |
| Lubricant | **1.5** |
| **TOTAL** | **100.0** |
| | |

| **COMPONENTS** | **Percentage formula (%)** |
|---|---|
| Coated fexofenadine granule (formula 5) | **54** |
| Mannitol | **28** |
| Crospovidone (Kollidon CL) | **5** |
| Sweetener | **1.5** |
| Effervescent agents | **5** |
| Microcrystalline cellulose | **5.0** |
| Lubricant | **1.50** |
| **TOTAL** | **100.00** |
| Coated fexofenadine granule (formula 5) | **54** |
| Mannitol | **28** |
| Crospovidone (Kollidon CL) | **5** |
| Sweetener | **1.5** |
| Effervescent agents | **5** |
| Flavourings | **0.5** |
| Microcrystalline cellulose | **4.5** |
| Lubricant | **1.5** |
| **TOTAL** | **100.0** |

For these three formulae, all the excipients of the external phase are mixed with the coated granules. The whole is then tableted.

### 4. Results of the tests performed

The protocols used for obtaining the results below are as described previously.

| **Formula** | **Disintegration time (s)** | **Mouthfeel** | **Observations** |
|---|---|---|---|
| Formula 1 | NA | Granular | Slight bitterness |
| Formula 2 | NA | Granular | Sliqht bitterness |
| Formula 3 | NA | Granular | Sliqht bitterness |
| Formula 4 | NA | Granular | Slight bitterness |
| Formula 5 | NA | Granular | Sliqht bitterness |
| Formula 6 | NA | Granular | No bitterness |
| Product 1 | NA | Rapid wetting, granular | Persistent, fresh pleasant taste* |
| Product 2 | NA | Rapid wetting, granular | Very pleasant, persistent, fresh taste* |

| | | | |
|---|---|---|---|
| *: Variable taste perception according to the type of flavourings used | | | |

- Dissolution profiles of products 1, 2 and tablets Telfast® (Allegra®) 180 mg are collated in the following tables.

| **Product 2** | | |
|---|---|---|
| **Time (min)** | **Mean** | **CV** |
| 0 | 0 | 0 |
| 5 | 43 | 18.5 |
| 10 | 58 | 11.6 |
| 15 | 65 | 9.5 |
| 30 | 74 | 6.1 |
| 45 | 78 | 4.7 |
| 60 | 80 | 4.5 |
| | | |

| **Product 1** | | |
|---|---|---|
| **Time (min)** | **Mean** | **CV** |
| **0** | 0 | NA |
| **5** | 66 | 2.5 |
| **10** | 80 | 1.2 |
| **15** | 87 | 1.2 |
| **30** | 93 | 1.3 |
| **45** | 95 | 1.2 |
| **60** | 96 | 0.9 |
| | | |

| **Telfast (or Allegra) ® 180 mg** | | |
|---|---|---|
| **Time (min)** | **Mean** | **CV** |
| 0 | 0 | NA |
| 5 | 71 | 4.1 |
| 10 | 85 | 2.5 |
| 15 | 90 | 2.4 |
| 30 | 95 | 0.9 |
| 45 | 97 | 1.3 |
| 60 | 97 | 1.6 |

| | | |
|---|---|---|
| "CV": represents the Coefficient of Variation, which is the ratio of the standard deviation to the mean obtained. | | |

### Conclusions

It may be noted that the addition of an adjuvant to the formulation in the fatty matrix, such as phospholipid (lecithin) added to a proportion of a few per cent, for instance from 1% to 3%, makes it possible to accelerate the dissolution.

In the absence of lecithin, a slowed dissolution profile is observed, the presence of 1.5% makes it possible to improve the dissolution kinetics and increasing the proportion to 5% does not afford any improvement. The optimum proportion in the specific example studied is of the order of 1.5%.

It may also be noted that the addition of a diluent also acting as permeabilizer to the formulation during granulation (step E1), added to a proportion of a few per cent, for instance from 3% to 10%, makes it possible to accelerate the dissolution. The addition of corn starch as diluent or permeabilizer thus improves the dissolution kinetics. The addition of 10% starch does not afford a better result than at 5%. The addition of this agent is preferable during step E1 (granulation) compared with the introduction of this adjuvant to the coating solution.

## Claims

1. Fexofenadine composition comprising (a) a granule centre formed from active principle grains aggregated in the presence of a binder and optionally of a lubricant or preferably of a diluent, and (b) a layer for coating the said granule centre formed from a fatty matrix, in which composition
• the fexofenadine represents at least 10%, preferably 15% or even 20% by weight relative to the weight of the composition; the active principle represents not more than 90%, preferably not more than 60% or 50% by weight relative to the weight of the composition, or even less than 40% by weight relative to the weight of the composition, preferably the active principle represents from 20% to 40% relative to the weight of the composition;
• the fatty matrix represents from 50% to 85% by weight of the composition, the fatty matrix optionally comprising an adjuvant, preferably chosen from hydrophilic agents, surfactants or mixtures thereof, and these adjuvants representing less than 10% by weight of the composition and preferably from 1% to 3% by weight relative to the weight of the composition;
• the binder, preferably a hydrophilic agent chosen from hydrophilic polymers and hot-melt agents, represents from 0.2% to 18% by weight relative to the weight of the composition, preferably the binder represents less than 18% by weight for a hot-melt agent, or even, preferably, the binder represents less than 10% by weight for a hydrophilic polymer relative to the weight of the composition;
• the diluent, if necessary, as filler represents a content of from 0 to 78.8%, preferably from 0 to 39% and preferably from 5% to 15% by weight relative to the weight of the composition;
• the lubricant, if necessary, as flow agent represents a content of from 0 to 1.8% relative to the weight of the composition.

2. Fexofenadine composition according to Claim 1, in which the said fatty matrix is formed from C14 to C22 and preferably C16 to C18 long-carbon-chain saturated fatty acids, pure or as mixtures, and/or the corresponding fatty alcohols thereof, and the binder is chosen from hydrophilic polymers.

3. Fexofenadine composition according to Claim 2, in which the said fatty matrix is formed from stearic acid.

4. Fexofenadine composition according to one of Claims 1, 2 and 3, in which the said fatty matrix comprises an adjuvant chosen from the group of surfactants (phospholipid, polysorbate, lauryl sulfate), hydrophilic excipients such as sucrose, polyols, cellulose, lactose, silica, dicalcium phosphate, carbonates, starch, macrogols, agents that are soluble at acidic pH (methacrylic derivatives), pure or as mixtures, preferably phospholipids.

5. Fexofenadine composition according to Claim 4, in which the adjuvant is soya lecithin.

6. Fexofenadine composition according to one of Claims 1 to 5, in which the adjuvant is a surfactant that represents less than 10% by weight, preferably less than 5% by weight, preferably from 1% to 3%, or even from 1% to 2%, by weight relative to the weight of the composition.

7. Fexofenadine composition according to one of Claims 1 to 6, in which the binder is povidone and the diluent is corn starch.

8. Fexofenadine composition according to one of Claims 1 to 7, in which the amount of fexofenadine represents more than 10% by weight and ranges up to 90% by weight relative to the weight of the composition.

9. Fexofenadine composition according to one of Claims 1 to 8, in which the amount of fexofenadine represents more than 10% by weight of the composition and the fatty matrix comprises an adjuvant.

10. Process for preparing a fexofenadine composition as defined according to one of Claims 1 to 9, the said process comprising the steps of:
E1) preparation of the granule centre by spraying an aqueous solution, an organic solution or a mixture thereof comprising a binder or by spraying a molten binder onto the fexofenadine alone or as a mixture with a lubricant and/or preferably a diluent;
E2) coating by spraying onto the granules a fatty matrix melted beforehand in a melting vessel at a temperature from about 10 to 20°C above its melting point;
E3) cooling of the composition obtained.

11. Process for preparing a composition according to Claim 10, in which:
• fexofenadine represents at least 10% and not more than 90%, preferably from 20% to 50%.

12. Process for preparing a composition according to Claim 10, in which:
• fexofenadine represents less than 40% by weight, preferably from 20% to 40% and advantageously from 30% to 40% relative to the weight of the composition, and
• the fatty matrix represents from 50% to 60% by weight of the composition.

13. Process according to one of Claims 10 to 12, in which the size of the coated granules obtained after step E3) is less than 500 µm, preferably less than 350 µm, preferably ranging from 50 to 350 µm.

14. Process according to one of Claims 10 to 13, in which the particle size of the final product obtained at step E3) is distributed according to the following range:
• less than 15% by weight of the coated granules are greater than 500 µm;
• more than 80% by weight, preferably more than 90% by weight, of the coated granules are between 350 and 50 µm, and;
• less than 20% by weight, preferably less than 5% by weight, of the coated granules are less than 50 µm.

15. Process according to one of Claims 10 to 14, in which the aqueous or organic solution used in step E1 comprises, as binder, a hydrophilic polymer, preferably chosen from the group of cellulose derivatives (hydroxypropylcellulose or ethylcellulose), povidone (polyvinylpyrrolidone), sucrose, gums, starches, gelatin, macrogols (polyethylene glycols), which represent approximately from 15% to 45% and preferably 20% to 40% by weight of the said solution.

16. Process according to one of Claims 10 to 15, in which the aqueous or organic solution used in step E1 sprayed onto the fexofenadine is mixed with corn starch.

17. Process according to one of Claims 10 to 16, in which the weight percentage of the binder constituting the coating of the granule obtained in step E1 represents from 1% to 7% by weight relative to the weight of the composition for a hydrophilic polymer.

18. Process according to one of Claims 10 to 17, followed by a step E4 of formulation of the coated granules obtained in step E3 with excipients such as diluents, fillers, viscosity modifiers, disintegrants, dyes, sweeteners, salivating agents, flavourings, preserving agents, wetting agents, effervescent agents, lubricants, buffers and sequestrants for the manufacture of an oral formulation in the form of a composition of granules to be swallowed for sachets, granules for a drinkable suspension, granules for tablets or granules for orodispersible tablets.

19. Fexofenadine composition that may be obtained according to the process described in one of Claims 10 to 18.

20. Pharmaceutical composition comprising the fexofenadine composition as described in Claims 1 to 9 or as may be prepared according to the process as described in Claims 10 to 18.

21. Composition according to Claim 20, **characterized in that** it is in the form of a sachet comprising a composition of granules to be swallowed or a sachet for a drinkable suspension and **characterized in that** it contains one or more excipients, preferably chosen from diluents, viscosity modifiers, sequestrants, buffers, preserving agents, lubricants, wetting agents, effervescent agents, dyes, sweeteners, salivating agents and flavourings, and mixtures thereof.

22. Composition according to Claim 20, **characterized in that** it is in the form of tablets, to be chewed, swallowed or sucked, or orodispersible tablets with masked taste, and **characterized in that** it contains one or more excipients, preferably chosen from diluents, binders, lubricants, salivating agents, anaesthetics, wetting agents, preserving agents, disintegrants, dyes, sweeteners, flavourings, and mixtures thereof.

23. Sachet or tablet according to Claim 21 or 22, **characterized in that** the weight ratio of the fexofenadine composition as defined according to one of Claims 1 to 9 relative to the weight amount of excipient in the sachet or tablet ranges from 0.2 to 0.8.

24. Use of a fexofenadine composition as defined in one of Claims 1 to 9 or prepared according to the process of any one of Claims 10 to 18, for the preparation of a pharmaceutical composition that is in the form of sachets comprising a composition of granules to be swallowed or for a drinkable suspension, tablets to be chewed, tablets to be swallowed, tablets to be sucked, or orodispersible tablets with masked taste.

## Patentansprüche

1. Fexofenadin-Zusammensetzung, umfassend (a) ein Granulatzentrum aus Wirkstoffkörnchen, die in Gegenwart eines Bindemittels und gegebenenfalls eines Gleitmittels oder vorzugsweise eines Verdünnungsmittels aggregiert worden sind, und (b) eine Schicht als Überzug für das Granulatzentrum aus einer Fettmatrix, wobei in der Zusammensetzung
• Fexofenadin mindestens 10 Gew.-%, vorzugsweise 15 Gew.-% oder sogar 20 Gew.-%, bezogen auf das Gewicht der Zusammensetzung, ausmacht, der Wirkstoff nicht mehr als 90 Gew.-%, vorzugsweise nicht mehr als 60 Gew.-% oder 50 Gew.-%, bezogen auf das Gewicht der Zusammensetzung, oder sogar weniger als 40 Gew.-%, bezogen auf das Gewicht der Zusammensetzung, ausmacht, vorzugsweise der Wirkstoff von 20 Gew.-% bis 40 Gew.-%, bezogen auf das Gewicht der Zusammensetzung, ausmacht,
• die Fettmatrix von 50 Gew.-% bis 85 Gew.-%, bezogen auf das Gewicht der Zusammensetzung, ausmacht, wobei die Fettmatrix gegebenenfalls ein Adjuvans umfasst, das vorzugsweise aus hydrophilen Mitteln, oberflächenaktiven Mitteln oder Gemischen davon ausgewählt wird, und diese Adjuvantien weniger als 10 Gew.-%, bezogen auf das Gewicht der Zusammensetzung, und vorzugsweise von 1 Gew.-% bis 3 Gew.-%, bezogen auf das Gewicht der Zusammensetzung, ausmachen,
• das Bindemittel, vorzugsweise ein aus hydrophilen Polymeren und Schmelzklebemitteln ausgewähltes hydrophiles Mittel, von 0,2 Gew.-% bis 18 Gew.-%, bezogen auf das Gewicht der Zusammensetzung, ausmacht, vorzugsweise das Bindemittel weniger als 18 Gew.-% im Fall eines Schmelzklebemittels ausmacht oder bevorzugt das Bindemittel sogar weniger als 10 Gew.-% im Fall eines hydrophilen Polymers, bezogen auf das Gewicht der Zusammensetzung, ausmacht,
• das Verdünnungsmittel, falls benötigt, als Füllstoff einen Gehalt von 0 bis 78,8 Gew.-%, vorzugsweise von 0 bis 39 Gew.-% und vorzugsweise von 5 Gew.-% bis 15 Gew.-%, bezogen auf das Gewicht der Zusammensetzung, ausmacht,
• das Gleitmittel, falls benötigt, als Fließmittel einen Gehalt von 0 bis 1,8 %, bezogen auf das Gewicht der Zusammensetzung, ausmacht.

2. Fexofenadin-Zusammensetzung nach Anspruch 1, wobei die Fettmatrix aus gesättigten C14- bis C22- und vorzugsweise C16- bis C18-Fettsäuren mit langen Kohlenstoffketten, rein oder als Gemische, und/oder deren entsprechenden Fettalkoholen hergestellt ist und das Bindemittel aus hydrophilen Polymeren ausgewählt ist.

3. Fexofenadin-Zusammensetzung nach Anspruch 2, wobei die Fettmatrix aus Stearinsäure hergestellt ist.

4. Fexofenadin-Zusammensetzung nach einem der Ansprüche 1, 2 und 3, wobei die Fettmatrix ein Adjuvans umfasst, das aus der Gruppe der oberflächenaktiven Mittel (Phospholipid, Polysorbat, Laurylsulfat), hydrophilen Excipienten, wie Saccharose, Polyole, Cellulose, Lactose, Siliziumdioxid, Calciumhydrogenphosphat, Carbonate, Stärke, Macrogole, Mitteln, die bei saurem pH-Wert löslich sind (Methacrylsäure-Derivate), rein oder als Gemische, vorzugsweise Phospholipiden, ausgewählt ist.

5. Fexofenadin-Zusammensetzung nach Anspruch 4, wobei das Adjuvans Sojalecithin ist.

6. Fexofenadin-Zusammensetzung nach einem der Ansprüche 1 bis 5, wobei das Adjuvans ein oberflächenaktives Mittel ist, das weniger als 10 Gew.-%, vorzugsweise weniger als 5 Gew.-%, vorzugsweise von 1 Gew.-% bis 3 Gew.-% oder sogar von 1 Gew.-% bis 2 Gew.-%, bezogen auf das Gewicht der Zusammensetzung, ausmacht.

7. Fexofenadin-Zusammensetzung nach einem der Ansprüche 1 bis 6, wobei das Bindemittel Povidon ist und das Verdünnungsmittel Maisstärke ist.

8. Fexofenadin-Zusammensetzung nach einem der Ansprüche 1 bis 7, wobei die Menge an Fexofenadin mehr als 10 Gew.-% ausmacht und im Bereich bis zu 90 Gew.-%, bezogen auf das Gewicht der Zusammensetzung, liegt.

9. Fexofenadin-Zusammensetzung nach einem der Ansprüche 1 bis 8, wobei die Menge an Fexofenadin mehr als 10 Gew.-%, bezogen auf das Gewicht der Zusammensetzung, ausmacht und die Fettmatrix ein Adjuvans umfasst.

10. Verfahren zur Herstellung einer Fexofenadin-Zusammensetzung nach einem der Ansprüche 1 bis 9, wobei das Verfahren die folgenden Schritte umfasst:
E1) Herstellung des Granulatzentrums durch Aufsprühen einer wässrigen Lösung, einer organischen Lösung oder eines Gemischs davon, die/das ein Bindemittel umfasst, oder durch Aufsprühen eines schmelzflüssigen Bindemittels auf das Fexofenadin allein oder als Gemisch mit einem Gleitmittel und/oder vorzugsweise einem Verdünnungsmittel,
E2) Überziehen der Granulate durch Aufsprühen mit einer Fettmatrix, die zuvor in einem Schmelzgefäß bei einer Temperatur von etwa 10 bis 20 °C über ihrem Schmelzpunkt geschmolzen wurde,
E3) Abkühlen der erhaltenen Zusammensetzung.

11. Verfahren zur Herstellung einer Zusammensetzung nach Anspruch 10, wobei:
• Fexofenadin mindestens 10 % und nicht mehr als 90 %, vorzugsweise 20 % bis 50 %, ausmacht.

12. Verfahren zur Herstellung einer Zusammensetzung nach Anspruch 10, wobei:
• Fexofenadin weniger als 40 Gew.-%, vorzugsweise von 20 % bis 40 % und vorteilhafterweise von 30 % bis 40 %, bezogen auf das Gewicht der Zusammensetzung, ausmacht und
• die Fettmatrix von 50 Gew.-% bis 60 Gew.-%, bezogen auf das Gewicht der Zusammensetzung, ausmacht.

13. Verfahren nach einem der Ansprüche 10 bis 12, wobei die Größe der nach Schritt E3) erhaltenen überzogenen Granulate kleiner als 500 µm, vorzugsweise von weniger als 350 µm, vorzugsweise im Bereich von 50 bis 350 µm ist.

14. Verfahren nach einem der Ansprüche 10 bis 13, wobei die Partikelgröße des im Schritt E3) erhaltenen Endprodukts gemäß dem folgenden Bereich verteilt ist:
• weniger als 15 Gew.-% der überzogenen Granulate sind größer als 500 µm,
• mehr als 80 Gew.-%, vorzugsweise mehr als 90 Gew.-%, der überzogenen Granulate sind zwischen 350 und 50 µm und
• weniger als 20 Gew.-%, vorzugsweise weniger als 5 Gew.-%, der überzogenen Granulate sind kleiner als 50 µm.

15. Verfahren nach einem der Ansprüche 10 bis 14, wobei die in Schritt E1 verwendete wässrige oder organische Lösung als Bindemittel ein hydrophiles Polymer, das vorzugsweise aus der Gruppe mit Cellulosederivaten (Hydroxypropylcellulose oder Ethylcellulose), Povidon (Polyvinylpyrrolidon), Saccharose, Gummen, Stärken, Gelatine, Makrogolen (Polyethylenglykolen) ausgewählt wird, die von etwa 15 Gew.-% bis 45 Gew.-% und vorzugsweise 20 Gew.-% bis 40 Gew.-%, bezogen auf das Gewicht der Lösung, ausmachen.

16. Verfahren nach einem der Ansprüche 10 bis 15, wobei die in Schritt E1 verwendete, auf das Fexofenadin aufgesprühte wässrige oder organische Lösung mit Maisstärke gemischt ist.

17. Verfahren nach einem der Ansprüche 10 bis 16, wobei der Gewichtsprozentsatz an Bindemittel, das den Überzug des in Schritt E1 erhaltenen Granulats bildet, von 1 Gew.-% bis 7 Gew.-%, bezogen auf das Gewicht der Zusammensetzung, im Fall eines hydrophilen Polymers ausmacht.

18. Verfahren nach einem der Ansprüche 10 bis 17, gefolgt von einem Schritt E4 der Formulierung der in Schritt E3 erhaltenen überzogenen Granulate mit Excipienten, wie Verdünnungsmitteln, Füllstoffen, Viskositätsmodifikatoren, Sprengmitteln, Farbstoffen, Süßstoffen, Speichel produzierenden Mitteln, Geschmacksstoffen, Konservierungsstoffen, Benetzungsmitteln, Brausemitteln, Gleitmitteln, Puffern und Sequestrierungsmitteln zur Herstellung einer oralen Formulierung in Form einer Zusammensetzung von Granulaten zum Schlucken für Portionsbeutel, Granulaten für eine trinkbare Suspension, Granulaten für Tabletten oder Granulaten für orodispergierbare Tabletten.

19. Fexofenadin-Zusammensetzung, die gemäß dem in einem der Ansprüche 10 bis 18 beschriebenen Verfahren erhältlich ist.

20. Pharmazeutische Zusammensetzung, die die Fexofenadin-Zusammensetzung nach einem der Ansprüche 1 bis 9 oder, wie sie gemäß dem Verfahren nach einem der Ansprüche 10 bis 18 hergestellt werden kann, umfasst.

21. Zusammensetzung nach Anspruch 20, **dadurch gekennzeichnet, dass** sie in Form eines Portionsbeutels, der eine Zusammensetzung von Granulaten zum Schlucken umfasst, oder eines Portionsbeutels für eine trinkbare Suspension vorliegt, und **dadurch gekennzeichnet, dass** sie einen oder mehrere Excipienten enthält, die vorzugsweise aus Verdünnungsmitteln, Viskositätsmodifikatoren, Sequestrierungsmitteln, Puffern, Konservierungsmitteln, Gleitmitteln, Benetzungsmitteln, Brausemitteln, Farbstoffen, Süßstoffen, Speichel produzierenden Mitteln und Geschmacksstoffen und Gemischen davon ausgewählt sind.

22. Zusammensetzung nach Anspruch 20, **dadurch gekennzeichnet, dass** sie in Form von Tabletten zum Kauen, Schlucken oder Lutschen oder orodispergierbaren Tabletten mit maskiertem Geschmack vorliegt, und **dadurch gekennzeichnet, dass** sie einen oder mehrere Excipienten enthält, die vorzugsweise aus Verdünnungsmitteln, Bindemitteln, Gleitmitteln, Speichel produzierenden Mitteln, Anästhetika, Benetzungsmitteln, Konservierungsmitteln, Sprengmitteln, Farbstoffen, Süßstoffen, Geschmacksstoffen und Gemischen davon ausgewählt sind.

23. Portionsbeutel oder Tablette nach Anspruch 21 oder 22, **dadurch gekennzeichnet, dass** das Gewichtsverhältnis der Fexofenadin-Zusammensetzung nach einem der Ansprüche 1 bis 9, bezogen auf das Gewicht des Excipienten in dem Portionsbeutel oder der Tablette im Bereich von 0,2 bis 0,8 liegt.

24. Verwendung einer Fexofenadin-Zusammensetzung nach einem der Ansprüche 1 bis 9 oder hergestellt gemäß dem Verfahren nach einem der Ansprüche 10 bis 18 zur Herstellung einer pharmazeutischen Zusammensetzung, die in Form von Portionsbeuteln, die eine Zusammensetzung von Granulaten zum Schlucken oder für eine trinkbare Suspension umfassen, Tabletten zum Kauen, Tabletten zum Schlucken, Tabletten zum Lutschen oder orodispergierbaren Tabletten mit maskiertem Geschmack vorliegt.

## Revendications

1. Composition de fexofénadine comprenant (a) un centre de granule formé de grains de substance active agrégés en présence d'un liant et facultativement d'un lubrifiant ou de préférence d'un diluant, et (b) une couche pour enrober ledit centre de granule formé d'une matrice grasse, composition dans laquelle
• la fexofénadine représente au moins 10 %, de préférence 15 % ou même 20 % en poids par rapport au poids de la composition ; la substance active représente pas plus de 90 %, de préférence pas plus de 60 % ou 50 % en poids par rapport au poids de la composition, ou même moins de 40 % en poids par rapport au poids de la composition, de préférence la substance active représente de 20 % à 40 % par rapport au poids de la composition ;
• la matrice grasse représente de 50 % à 85 % en poids de la composition, la matrice grasse comprenant facultativement un adjuvant, de préférence choisi parmi agents hydrophiles, des tensioactifs ou des mélanges de ceux-ci, et ces adjuvants représentant moins de 10 % en poids de la composition et de préférence de 1 % à 3 % en poids par rapport au poids de la composition ;
• le liant, de préférence un agent hydrophile choisi parmi des polymères hydrophiles et des agents de fusion, représente de 0,2 % à 18 % en poids par rapport au poids de la composition, de préférence le liant représente moins de 18 % en poids pour un agent de fusion, ou même, de préférence, le liant représente moins de 10 % en poids pour un polymère hydrophile par rapport au poids de la composition ;
• le diluant, si nécessaire, en tant que charge représente une teneur de 0 à 78,8 %, de préférence de 0 à 39 % et de préférence de 5 % à 15 % en poids par rapport au poids de la composition ;
• le lubrifiant, si nécessaire, en tant qu'agent d'écoulement représente une teneur de 0 à 1,8 % par rapport au poids de la composition.

2. Composition de fexofénadine selon la revendication 1, dans laquelle ladite matrice grasse est formée d'acides gras saturés à chaîne de carbone longue de C14 à C22 et de préférence de C16 à C18, purs ou sous forme de mélanges, et/ou les alcools gras correspondants de ceux-ci, et le liant est choisi parmi des polymères hydrophiles.

3. Composition de fexofénadine selon la revendication 2, dans laquelle ladite matrice grasse est formée d'acide stéarique.

4. Composition de fexofénadine selon une des revendications 1, 2 et 3, dans laquelle ladite matrice grasse comprend un adjuvant choisi dans le groupe de tensioactifs (phospholipide, polysorbate, sulfate de lauryle), excipients hydrophiles tels que saccharose, polyols, cellulose, lactose, silice, phosphate dicalcique, carbonates, amidon, macrogols, agents qui sont solubles à pH acide (dérivés méthacryliques), purs ou sous forme de mélanges, de préférence des phospholipides.

5. Composition de fexofénadine selon la revendication 4, dans laquelle l'adjuvant est la lécithine de soja.

6. Composition de fexofénadine selon une des revendications 1 à 5, dans laquelle l'adjuvant est un tensioactif qui représente moins de 10 % en poids, de préférence moins de 5 % en poids, de préférence de 1 % à 3 %, ou même de 1 % à 2 %, en poids par rapport au poids de la composition.

7. Composition de fexofénadine selon une des revendications 1 à 6, dans laquelle le liant est la povidone et le diluant est l'amidon de maïs.

8. Composition de fexofénadine selon une des revendications 1 à 7, dans laquelle la quantité de fexofénadine représente plus de 10 % en poids et peut aller jusqu' à 90 % en poids par rapport au poids de la composition.

9. Composition de fexofénadine selon une des revendications 1 à 8, dans laquelle la quantité de fexofénadine représente plus de 10 % en poids de la composition et la matrice grasse comprend un adjuvant.

10. Procédé pour préparer une composition de fexofénadine telle que définie selon une des revendications 1 à 9, ledit procédé comprenant les étapes de :
E1) préparation du centre de granule par pulvérisation d'une solution aqueuse, une solution organique ou un mélange de celles-ci comprenant un liant ou par pulvérisation d'un liant fondu sur la fexofénadine seule ou sous forme de mélange avec un lubrifiant et/ou de préférence un diluant ;
E2) enrobage par pulvérisation sur les granules d'une matrice grasse préalablement fondue dans une cuve de fusion à une température d'environ 10 à 20 °C au-dessus de son point de fusion ;
E3) refroidissement de la composition obtenue.

11. Procédé pour préparer une composition selon la revendication 10, dans lequel :
• la fexofénadine représente au moins 10 % et pas plus de 90 %, de préférence de 20 % à 50 %.

12. Procédé pour préparer une composition selon la revendication 10, dans lequel :
• la fexofénadine représente moins de 40 % en poids, de préférence de 20 % à 40 % et avantageusement de 30 % à 40 % par rapport au poids de la composition, et
• la matrice grasse représente de 50 % à 60 % en poids de la composition.

13. Procédé selon une des revendications 10 à 12, dans lequel la taille des granules enrobés obtenus après l'étape E3) est inférieure à 500 µm, de préférence inférieure à 350 µm, de préférence dans la plage de 50 à 350 µm.

14. Procédé selon une des revendications 10 à 13, dans lequel la taille de particule du produit final obtenu à l'étape E3) est distribuée selon la plage suivants :
• moins de 15 % en poids des granules enrobés sont supérieurs à 500 µm ;
• plus de 80 % en poids, de préférence plus de 90 % en poids, des granules enrobés sont compris entre 350 et 50 µm, et ;
• moins de 20 % en poids, de préférence moins de 5 % en poids, des granules enrobés font moins de 50 µm.

15. Procédé selon une des revendications 10 à 14, dans lequel la solution aqueuse ou organique utilisée dans l'étape E1 comprend, en tant que liant, un polymère hydrophile, de préférence choisi dans le groupe de dérivés de cellulose (hydroxypropylcellulose ou éthylcellulose), povidone (polyvinylpyrrolidone), saccharose, gommes, amidons, gélatine, macrogols (polyéthylèneglycols), qui représentent approximativement de 15 % à 45 % et de préférence de 20 % à 40 % en poids de ladite solution.

16. Procédé selon une des revendications 10 à 15, dans lequel la solution aqueuse ou organique utilisée dans l'étape E1 pulvérisée sur la fexofénadine est mélangée avec de l'amidon de maïs.

17. Procédé selon une des revendications 10 à 16, dans lequel le pourcentage en poids du liant constituant l'enrobage du granule obtenu dans l'étape E1 représente de 1 % à 7 % en poids par rapport au poids de la composition pour un polymère hydrophile.

18. Procédé selon une des revendications 10 à 17, suivi par une étape E4 de formulation des granules enrobés obtenus dans l'étape E3 avec des excipients tels que des diluants, des charges, des modificateurs de viscosité, des délitants, des colorants, des édulcorants, des agents de salivation, des arômes, des agents conservateurs, des agents mouillants, des agents effervescents, des lubrifiants, des tampons et des séquestrants pour la fabrication d'une formulation orale sous la forme d'une composition de granules à avaler pour sachets, de granules pour une suspension buvable, de granules pour des comprimés ou granules pour des comprimés orodispersibles.

19. Composition de fexofénadine qui peut être obtenue selon le procédé décrit dans une des revendications 10 à 18.

20. Composition pharmaceutique comprenant la composition de fexofénadine telle que décrite dans les revendications 1 à 9 ou telle qu'elle peut être préparée selon le procédé tel que décrit dans les revendications 10 à 18.

21. Composition selon la revendication 20, **caractérisée en ce qu'**elle est sous la forme d'un sachet comprenant une composition de granules à avaler ou un sachet pour une suspension buvable et **caractérisée en ce qu'**elle contient un ou plusieurs excipients, de préférence choisis parmi des diluants, modificateurs de viscosité, des séquestrants, des tampons, des agents conservateurs, des lubrifiants, des agents mouillants, des agents effervescents, des colorants, des édulcorants, des agents de salivation et des arômes, et des mélanges de ceux-ci.

22. Composition selon la revendication 20, **caractérisée en ce qu'**elle est sous la forme de comprimés, à mâcher, avaler ou sucer, ou des comprimés orodispersibles avec un goût masqué, et **caractérisée en ce qu'**elle contient un ou plusieurs excipients, de préférence choisis parmi des diluants, des liants, de lubrifiants, des agents de salivation, des anesthésiques, des agents mouillants, des agents conservateurs, des délitants, des colorants, des édulcorants, des arômes, et des mélanges de ceux-ci.

23. Sachet ou comprimé selon la revendication 21 ou 22, **caractérisé en ce que** le rapport en poids de la composition de fexofénadine telle que définie selon une des revendications 1 à 9 par rapport à la quantité en poids d'excipient dans le sachet ou comprimé est dans la plage de 0,2 à 0,8.

24. Utilisation d'une composition de fexofénadine telle que définie dans une des revendications 1 à 9 ou préparée selon le procédé de l'une quelconque des revendications 10 à 18, pour la préparation d'une composition pharmaceutique qui est sous la forme de sachets comprenant une composition de granules à avaler ou pour une suspension buvable, des comprimés à mâcher, des comprimés à avaler, des comprimés à sucer, ou des comprimés orodispersibles avec un goût masqué.
